Europäisches Patentamt

European Patent Office

Office européen des brevets

(11) Publication number: **0 254 430 B1**

# EUROPEAN PATENT SPECIFICATION

(45) Date of publication of patent specification: **08.09.93**

(51) Int. Cl.5: **G01N 33/543**, G01N 21/47, //G01N33/557,G01N33/76, G01N33/78,G01N33/94, G01N33/80

(21) Application number: **87305669.1**

(22) Date of filing: **25.06.87**

(54) **Light-scattering immunoassay system.**

(30) Priority: **26.06.86 US 879236**

(43) Date of publication of application:
**27.01.88 Bulletin 88/04**

(45) Publication of the grant of the patent:
**08.09.93 Bulletin 93/36**

(84) Designated Contracting States:
**AT BE CH DE FR GB IT LI LU NL SE**

(56) References cited:
| | |
|---|---|
| **EP-A- 0 075 353** | **EP-A- 0 091 636** |
| **EP-A- 0 167 335** | **EP-A- 0 170 376** |
| **EP-A- 0 753 535** | **US-A- 3 939 350** |

**JOURNAL OF CHROMATOGRAPHY, vol. 376, April 1986, Amsterdam (NL); C.J. GRIBNAU et al., pp. 175-189&NUM;**

(73) Proprietor: **ORTHO DIAGNOSTIC SYSTEMS INC.**
**One Johnson & Johnson Plaza**
**New Brunswick New Jersey 08933-7003(US)**

(72) Inventor: **Schutt, Ernest G.**
**7 Ouail Run**
**Long Valley, NJ 07853(US)**
Inventor: **Dondero, Richard S.**
**37 Hillside Avenue**
**Riverdale, NJ 07457(US)**

(74) Representative: **Jones, Alan John et al**
**CARPMAELS & RANSFORD 43 Bloomsbury Square**
**London, WC1A 2RA (GB)**

## Description

Field of the Invention

This invention relates to immunoassays generally and more particularly provides a new system for performing virtual homogeneous immunoassays employing colloidal gold.

Background of the Invention

Many human disease states are identified on the basis of immunoassay techniques which rely upon the specificity between antibodies and antigens, or ligand binding partners and ligands respectively and interchangeably. Over the past fifteen or so years, there has been a substantial amount of effort involved in the development of immunoassay techniques utilizing the so-called sandwich and competitive techniques. The sandwich technique involves the immobilization of an antigen by one antibody and then subsequent labeling by attachment of a second antibody having associated therewith a detectable label. Reverse immunoassays for the detection of antibody are similar but instead put antigen on the surface for reaction with the sample antibody. Competitive techniques are useful for antigens having only a single epitopic site for reaction with an antibody. Accordingly, and as the name implies, such techniques rely upon the competition of the antigen with another labeled antigen for a binding site on an immobilized antibody. The substitutions necessary for antibody detection tests are obvious and need not be covered here in any great detail.

Of great importance in the laboratory is the development of highly sensitive techniques which can be run in either batch random access, panel, or stat modes. Preferably, such techniques will be homogeneous in nature, i.e., and as used herein, they will be conducted solely within one container without any accompanying requirement to physically separate out components following reactions during the assay.

It is one object of the present invention to provide a new immunoassay system which is highly sensitive and which is homogeneous in nature.

U.S. Patent 3,939,350 to Kronick and the Kronick citations therein referenced describe an immunoassay system which allows for the measurement of biochemical analytes by fluorescence in a liquid sample. Kronick employs a physical phenomenon known under the name of total internal reflectance. This optical phenomenon occurs wherein light, when directed through a high refractive index material toward the interface of that material with a second material having a lower refractive index at greater than a critical angle, all light is reflected from that interface save for a microscopic evanescent wave which propagates into the second material for only a short distance. The second material may, for instance, be water or other aqueous medium in which an assay is being conducted. Kronick noted that when he brought materials which had been fluorescently labeled down to the interface and within the field of the evanescent wave, he could energize the fluorescent molecules and detect fluorescence which then emanated into the overlying solution. The Kronick system, however, looks at fluorescence which cannot be readily modified by alteration of the fluorescent labels in order to suit the system under study. Due to the nature of the specificity of the fluorescent label with respect to the wavelength of the excitation frequency, one is limited to a discrete light source providing the critical excitation frequency. To date, most investigators favor the He-Ne laser light source due to its reliability and low cost as well as the low cost of associated optics. Such a light source, however, brings concomitant difficulties in tailoring fluorescent molecules to be excited by the He-Ne laser output. The organic, inorganic, and bio-organic techniques required are especially difficult to control in the immunoassay arena. Further, Kronick's reliance on fluorescence is accompanied by additional disadvantages associated with bleaching of the fluorescent molecules and generally critical matching of fluorescent molecule excitation wavelength with laser output wavelength necessary to obtain good quantum efficiency.

It is an object of the present invention to provide a new immunoassay system which avoids the disadvantages associated with fluorescent labels and the criticality associated with matching an excitation source.

It is another object of the present invention to employ the principles of total internal reflection but with far greater flexibility regarding the choice of illumination sources.

U.S. Patent 4,181,441 to Noller describes a system similar to that of Kronick. Noller, however, taught that the assay should be conducted by measurement of light absorption in a liquid sample which could then be correlated to the presence of biochemical analytes. Although the Noller system employs different physical principles than the Kronick system, light absorption measurements are similarly subject to poorer signal-to-noise ratios due to small differences in large light signals thereby making such a system inherently less sensitive than desired.

It is another object of the present invention to avoid employing light absorption measurements while still gaining the advantages to be provided by the total internal reflectance phenomenon.

U.S. Patent 4,521,522 to Lundstrom teaches yet another immunoassay based upon reflectance and the use of Brewster's angle. This system relies upon a different optical phenomenon wherein directing a light beam, polarized in the plane of incidence, upon an interface, for example that formed between plastic and liquid, results in the transmission of a strong light beam into the liquid when such light strikes the interface at the Brewster angle. At the Brewster angle, substantially no light is reflected.

The Brewster angle is a function of the refractive indices of the two materials as well as the direction of polarization. Lundstrom noted that upon the growth of a biochemical layer at the interface, the Brewster angle condition would be disrupted resulting in increasing light reflectance, particularly at angles less than the Brewster angle. Unfortunately, the Lundstrom assay only works effectively with a wash step since the transmission of the beam into the liquid also results in the generation of light scatter and thus a spurious signal.

It is another object of the present invention to utilize light scatter but to avoid light scatter generated by the transmission of light into the liquid which occurs naturally when light is directed at an interface at the Brewster angle. Accordingly, it is yet another object of the present invention to avoid employing a Brewster angle condition.

EP-A-0170376 discloses a photometric instrument for use in the optical analysis of test samples possessing light scattering properties. The scattered light that is to be detected and analysed is collected at angles greater than 90° to the original light source.

EP-A-0075353 also discloses a photometric instrument for use in the optical analysis of test samples possessing light scattering properties. The scattered light that is to be detected and analysed is collected by means of a detector that is positioned opposite the original light source.

EP-A-0167335 discloses a method for observing the binding of an antibody to a surface-immobilised antigen by utilising the Bragg scattering angle.

According to a first aspect of the present invention, there is provided an apparatus for use in an immunoassay system employing an immunologically active component labelled with a light scattering particle for the detection of a ligand or ligand binding partner in an aqueous solution comprising; a container for receiving the aqueous sample having a refractive index greater than that of the aqueous sample; a light source for providing illumination; optical coupling means for directing the illumination towards the interface between the container and the aqueous solution; and alignment means for receiving the cuvette in fixed relationship to the optical means and the light source; characterised in that the illumination is directed towards the interface between the container and the aqueous solution at an angle equal to or just greater than the critical angle, and the apparatus further comprises photodetector means positioned to receive light scattered by the light scattering particle back towards the light source.

According to a second aspect of the present invention there is provided an immunoassay method for the detection of a ligand or a ligand binding partner in an aqueous solution employing colloidal gold as a label in the immunoassay; characterised in that the immunoassay is conducted in the field of an evanescent wave of an illumination directed towards the ligand or ligand binding partner at an angle equal to or just greater than the critical angle, and wherein the method includes the steps of measuring light scattered by the presence of the colloidal gold particle in the evanescent field back towards the original illumination; and correlating the colloidal gold scattered light as a function of the presence of the ligand or ligand binding partner to be determined.

According to a third aspect of the present invention there is provided a method for determining the presence of a ligand in an aqueous sample suspected of containing the ligand comprising the steps of: providing a first ligand binding partner specific for the ligand in the aqueous sample, the first ligand binding partner being labelled with a particle having light scattering characteristics in the aqueous sample; combining the first ligand binding partner and the aqueous sample with one side of an optically transmissive material thereby forming an interface between the material and the sample, the material having a plurality of second ligand binding partners bound to the material in contact with the aqueous sample and capable of binding to the ligand, the optically transmissive material having a refractive index greater than the refractive index of the aqueous sample; illuminating the interface through the optically transmissive material at an angle substantially equal to the critical angle to provide total internal reflectance within the material; and measuring the amount of light scattered from the interface back towards the original illumination, such that the amount of scattered light is a function of the amount of ligand present in the assay medium.

In accordance with various aspects and the principles of the present invention, there is provided an immunoassay system which utilises scattered total internal reflectance (STIR) as a measure of the presence of particular ligands to be determined in an aqueous solution. The invention relies in part upon the identification of the critical angle associated with total internal reflectance. The angle is largely a function of the refractive index of the material through which an incident light wave is directed, e.g. plastic, and the relatively lower refractive index of the material in which the immunoassay is being conducted, e.g. an aqueous solution. It is measured from a line perpendicular to the interface between the two materials, and thus at its maximum, 90°, will lie in the plane of the interface.

Light directed through the plastic towards the interface formed by the aqueous sample and plastic materials at the critical angle will result in total internal reflectance of the light within the plastic. It is recognized that no materials in the real world are perfect and accordingly it is preferred that the incident light be directed towards the interface at an angle several degrees greater than the critical angle, most preferably in the range of approximately 6° greater in order to ensure that the basic conditions of total internal reflectance are met. At such an angle, the incident collimated light, preferably from a laser, is totally internally reflected within the plastic save for the propagation of the evanescent wave parallel to the surface of the plastic and approximately $\frac{1}{4}\lambda$. from the surface. Similarly, smooth surfaces at the interface are preferred for optimum signal quality. Unlike conventional fluorescent techniques including those of Kronick, the present assay system is flexible with respect to light wavelength since particle size may be readily adjusted to match the available light source (or vice versa) to provide acceptable light scatter. Fluorescent molecules are not readily adjustable with respect to excitation wavelength.

Most ideally, the light source will be a He-Ne light source, however, other lasers with different wavelength outputs have been used and still other sources suggest themselves including light emitting diodes and other nonlaser light sources.

Applicant's immunoassay system further relies upon conventional immunoassay techniques but with one important difference. Applicant ideally employs a particulate label having a higher refractive index than that of the solution and most preferably also higher than the first light transmissive material, e.g. plastic in the foregoing example. Such particles would include, for instance, red blood cells, other materials having a highly reflective surface such as metallic particles, and nonmetallic substances such as glass or plastics, e.g. latex particles, and the like. Most preferably, colloidal gold is used as a label for the solution phase immunologically active component. While colloidal gold as a label is known, see for example U.S. 4,313,734 to Leuvering, almost no nonagglutination related use of the label has been made to date due to the difficulties associated with detection, particularly in homogenous type systems. It was surprisingly discovered by the inventors hereof that the unique combination of STIR with colloidal gold has resulted in an extremely efficient and sensitive homogeneous assay system. It is believed, but not known for certain that this is due primarily to the interaction of the colloidal gold particles with the evanescent wave. Indeed, experience implies that particles having an increasingly higher index of refraction than that of the underlying solid generally leads to an increase in light scatter. While particles with indices of refraction less than the underlying solid, providing they are also not equal to that of the aqueous medium, would also scatter light, such are less preferred.

Assuming for the moment a conventional sandwich technique, one immunoglobulin or ligand binding partner is immobilized on the surface and binds antigen or other ligand to be determined. Thereafter, (or simultaneously, or if not previously) a second immunoglobulin, directed at a second epitopic site on the ligand, and labeled directly or indirectly with colloidal gold, binds to the ligand creating the so-called "sandwich". In this arrangement, the presence of the colloidal gold disrupts the propagation of the evanescent wave resulting in scattered light which may be detected by a photomultiplier or other light sensor to provide a responsive signal. Another important aspect of the present invention involves the physical location of the detector. The detector is ideally placed at an angle greater than the critical angle and in a location whereby only light scattered backward toward the light source is detected. This location thereby ideally avoids the detection of spurious scattered light within the bulk liquid medium.

Another feature of the instant invention is that the immunoassays are diffusion rate controlled and not particularly temperature dependent. This is in strong contrast to ELISA and various other immunoassay techniques wherein temperature control is critical since small changes in temperature in such systems results in wide variations in assay results per unit of time.

It was surprisingly found by the inventors hereof, that as a result of the combination of these elements, rapid, sensitive results could be obtained in a homogeneous environment without requiring the complicated equipment previously associated with colloidal gold assay techniques.

Detailed Description of the Invention and Best Mode

An apparatus embodying the principles of STIR was constructed utilizing an equilateral flint glass prism, Model 01-PES-007, obtained from Melles-Grio. The prism was mounted on a support with one side held horizontal. An antibody-coated cuvette in the form of a microtiter well, available from Dynatech under the trade name IMMUNOLON TWO (styrene) was optically coupled to the horizontal prism surface with standard microscope oil. A five miliwatt helium neon laser (Hughes 3225-H-PC) was used to illuminate part of the cuvette's bottom surface at an angle 6° past the critical angle. Optionally, a cylindrical lens may be used to assist in focusing the laser light beam.

The critical angle was first determined by filling an uncoated cuvette, optically mounted on the prism, with a scattering aqueous medium comprising a mixture of colloidal gold sol produced pursuant to the method reported in Scanning Electron Microscopy Vol II, 9-31 (1981), Sear Inc., AMF, O'Hare, Chicago, generating particle sizes of about 30 to 50 nm and serum. The prism was rotated along an axis transverse to the axis of the incident light until the laser beam path, visible inside the cuvette, optically disappeared indicating that substantially all of the incident light was being reflected at the cuvette-liquid interface, the internal reflectance phenomenon known to occur at the critical angle. This critical angle between a perpendicular through the surface having the optically mounted cuvette and the laser beam was measured, the prism was reinstalled to provide a horizontal surface and the laser adjusted to illuminate the surface internally through the prism at an angle equal to 6° plus the critical angle. While a polarized laser was used with its polarization aligned with the electric field parallel to the plane of the styrene liquid interface, such is merely preferred but not necessary. Indeed virtually any collimated illumination source will serve. Similarly, while a prism was convenient, any optical coupling device for directing illumination toward the aqueous solid interface may be used such that total internal reflectance can be achieved by that interface.

A photodetector (Hammamatzu No. G1742 photodiode) was positioned at an angle above the critical angle but less than 90° at a position physically near the laser such that it would detect light scattered back toward the laser. In this position, minimal laser light is detected prior to the assay despite imperfections present at the interface.

Thus, placement of the photodetector above the critical angle is believed very important in order to insure that light propagating through the solution, e.g. straight light or secondary light scatter induced by irrelevant sources, cannot reach the detector. As a related advantage, this greatly reduces the effect of the sample's color or turbidity and of bubbles present at the liquid interface.

The electrical signal from the photodetector was electrically coupled to a high gain current amplifier (Kiethly Electrometer 610-C) and the output recorded on a strip chart recorder or digitally recorded by a computer data acquisition system (HP controller 3497A with HP 9836 computer). Reaction rates were then graphically determined on the recorder chart or calculated conventionally employing the computer.

## Example 1 - hCG Sandwich Assay

An anti-hCG antibody-coated cuvette (coated by standard physical adsorption) was positioned on the oil-coated prism with a laser internally reflecting off the center of the cuvette. 35 $\mu$ls of assay buffer (0.01 M phosphate buffered saline at a pH of 7.4 containing 1% bovine serum albumin, 1 M NaCl and 1.5 mg/ml mouse IgG) was added to the cuvette. 50 $\mu$ls of nonblocking, anti-hCG antibody coupled with colloidal gold (approximately 44 nm in size) was then added and mixed by pipette aspiration. 25 $\mu$ls of serum sample or serum-based standard (Gilford) was then added to the cuvette and mixed. The intensity of the scatter signal was recorded by a strip chart recorder and by a digital data acquisition system. The reaction rate was permitted to equilibrate for the first five minutes to permit the system to become linear and then measured kinetically during the next five minutes. Reaction rates (e.g. signal slopes) of unknown serum samples were compared to the reaction rates of standards in order to compute hCG concentrations. The results were as follows:

| Std. | Signal Slope (arbitrary units) |
|------|-------------------------------|
| 0 mIU | 1.00 |
| 10 mIU | 7.43 |
| 25 mIU | 16.33 |
| 50 mIU | 32.03 |
| 100 mIU | 68.67 |
| 200 mIU | 130.97 |

Example 2 - Test For Antibody (Reverse hCG Sandwich Assay)

hCG antigen was coated onto Immunolon cuvettes and positioned on the oil-coated prism as in Example 1. 50 $\mu$ls of colloidal gold (approximately 45 nm) coated with hCG was added to the cuvette along with 35 $\mu$ls of assay buffer as described in Example 1, and mixed. 25 $\mu$ls of mouse monoclonal anti-hCG containing standard (diluted in pH 8.3 HEPES/TRIS 0.225 M +0.5% BSA) added and mixed. After a five-minute delay for equilibration, the rate was measured as in Example 1. As anti-hCG concentrations were increased up to 10 $\mu$gs per ml, increasing rates of light scatter were observed with rates decreasing above this concentration giving the expected hook effect (e.g. insufficient labeled and immobilized antigen to accomodate all of the antibody present). The data was:

| Mouse IgG Conc. (ng) | Signal Slope (arbitrary units) |
|----------------------|-------------------------------|
| 0 ng | 8.02 |
| 10 ng | 10.27 |
| 100 ng | 12.35 |
| 1 $\mu$g | 75.84 |
| 10 $\mu$g | 91.39 |
| 100 $\mu$g | 37.00 |

Example 3 - Competition with Antigen-Coated Cuvette

Thyroxin (T$_4$) was covalently coupled to BSA with approximately 20 T$_4$ molecules per BSA molecule employing the following procedure. T$_4$-BSA conjugate was prepared from coupling BSA with T$_4$MC, L-Thyroxinyl-4-(N-maleimido-methyl)-cyclohexane-1-carbonate, through a nucleophilic addition at pH 9-10 by amino groups of BSA to the maleimido group of T$_4$MC. T$_4$MC was derivatized from SMCC, succimimidyl-4-(N-maleimido-methyl)-cyclohexane-1-carboxylate (Pierce Chemical), with L-Thyroxine by amidation at neutral pH. T$_4$-BSA conjugate was absorbed to commercial, strip microtiter wells by incubating 0.1 mls of 0.17 mgs per ml of the conjugate in 0.01 M phosphate buffer adjusted to a pH of 7 at room temperature for 18 hours. The wells were washed three times with 0.25 M HEPES/TRIS buffer (containing 0.05% NaN$_3$, 0.15 M NaCl at a pH of 8.3). The wells were then incubated for 72 hours at room temperature with 0.2 mls of HEPES/TRIS buffer plus 1% BSA. The wells were then again washed three times with HEPES/TRIS buffer and stored with buffer at 4°C. until use.

Monoclonal anti-T$_4$ IgG was coated with colloidal gold having an average diameter of 40 nm by previously described methods. The strip well cuvettes were mounted on the prism as in Example 1 and 65 $\mu$ls of pH 7.4 PBS containing 0.02% NaN$_3$ and 2% bovine gammaglobulin was added to the cuvette followed by 10 $\mu$ls of T$_4$ standard in the same buffer. 25 $\mu$ls of anti-T$_4$ antibody coated colloidal gold was

6

then added to the cuvette and mixed. The reaction rate was measured after an equilibration period. As expected, increasing $T_4$ concentrations correlated with decreased signal rates from back scattered light signal as follows:

| $T_4$ (μg/dl) | Signal Slope (arbitrary units) |
| --- | --- |
| 0 | 51.1 |
| 2 | 41.9 |
| 4 | 25.3 |
| 8 | 9.08 |
| 12 | 6.51 |
| 24 | 2.96 |

Example 4 - Competition With Antigen-Coated Colloidal Gold

Immunolon strip well cuvettes were coated with 0.1 mls of 5 μgs per ml of anti-digoxin and 0.1 M $KPO_4$ at a pH 7.4 and stored at 4°C. until use. The wells were then washed three times with 0.01 M PBS at a pH 7.4. Colloidal gold particles having an average diameter of 40 nm were coated with 1 mg per ml of digoxin--BSA conjugate (approximately 5 digoxins per BSA molecule) by the method set forth in an article by T. W. Smith, Biochemistry 9:331-337 (1970) and then diluted 1 to 4. 35 μls of buffer (0.01 M PBS, 1.0 M NaCl, 1% BSA at pH 7.6) was added to the cuvette followed rapidly by the addition of 25 μls of serum samples or serum base standard and 50 μls of digoxin-coated colloidal gold suspension and mixed. The reaction rate was measured during the next five minutes and the results observed. Increasing digoxin concentrations resulted in reduced reaction rates as follows:

| Digoxin (μ/ml) | Signal Slope (arbitrary units, 2 runs) |
| --- | --- |
| 0 | 372, 296 |
| 0.25 | 127, 86 |
| 0.50 | 30, 29 |

Example 5 - Internalized Kinetic Calibrator

It will be recognized that there may be variation from well to well between assays as well as between liquid reagents added to the wells. These differences will result in variations in kinetic responses which could, without correction, lead to erroneous results. One preferred method of correction is to utilize an internalized kinetic calibrator. To do so, a low level control sample is added to the well at the beginning of every assay and the rate of reaction monitored for a short time prior to the addition of the sample to the same well. The control sample can thus be used to calibrate each individual well, e.g. measuring the well's sensitivity and using that information to correct the sample readings, thereby obviating differences in structural or reagent coating uniformity. Accordingly, homogeneous rate assays can be ideally performed by first adding a control sample and monitoring the level of detector output. As a related advantage, this procedure will eliminate the need to perform duplicate assays thereby saving in time and resource expenditures. Such a calibration procedure will also obviate the sample to sample variations in light scattering efficiency of the particles which is a strong function of the index by refraction of the individual sample. The following example of the procedure demonstrates the princples involved.

7

Molded polycarbonate cuvettes were adsorbtion coated with anti-hCG antibody. 150 $\mu$ls of assay buffer (from Example 1). 100 $\mu$ls of anti-hCG coated colloidal gold (approximately 40 nms diameter) and 75 $\mu$ls of Gilford stripped serum based 10 mIU/ml calibrator was added to each cuvette and mixed. After a 5 minute incubation, the rate of increase of scattered light intensity (slope) was measured during the next 5 minutes. After recording this calibration slope, 75 $\mu$ls of Gilford serum based standard was added as sample, mixed and incubated 5 minutes before reading the scattered light slope during the next 5 minutes. The net calibrated slope of each cuvette was calculated by the equation:

Net calibrated slope = [slope of standard/slope of calibrator] - 0.8826

Where 0.8826 was the average slope of six zero hCG standards divided by their respective calibration slopes.

The CV (coefficient of variation) of six replicates of the following standards were calculated on the basis of the net calibrated slope and compared to the uncorrected slope of these standards. The data was as follows:

| mIU/ml of standard | CV of uncorrected | CV of Net Calibrated slope |
|---|---|---|
| 10 mIU/ml | 18.31% | 10.79% |
| 50 mIU/ml | 30.3 % | 21.42% |
| 100 mIU/ml | 18.86% | 5.88% |
| 200 mIU/ml | 33.63% | 30.86% |

In all cases, it can be seen that greater accuracy and repeatability was obtained using the internal calibration method.

Example 6 - Competitive hCG Assay Using Latex Particles

Immulon strip wells were coated as stated in Example 1 above. 35 $\mu$ls of assay buffer was added to each well. 25 $\mu$ls of hCG dissolved in stripped serum (Gilford) was then added and mixed. After a 5 minute incubation, 50 $\mu$ls of "Ortho Beta-hCG Slide Test for Pregnancy" hCG coated styrene latex (0.375 micron diameter) (Ortho Diagnostic Systems Inc.) was added and mixed. The reaction rate was permitted to equilibrate for 5 minutes while the slope of the scattered light signal was calculated during the next 5 minutes. The results were as follows:

| HCG Standard Concentration | Signal Slope (arbitrary units) | Average |
|---|---|---|
| 223,875 mIU/ml | 3.61, 3.76, 6.04 | 4.47 |
| 22,387 | 8.96, 9.02, 9.25 | 9.08 |
| 2,238 | 118, 122, 144 | 128 |
| 223 | 158, 162, 187 | 169 |
| 22.3 | 148, 157, 196 | 167 |
| 2.2 | 138, 142, 161 | 147 |

Example 7 - Direct Red Cell Antigen Test using Red Cell Particle (approximately 8 micron diameter)

Polycarbonate cuvettes were coated by adsorbtion with anti-D (anti-Rh$_o$) for an RH factor test and with anti-A for an ABO blood group test. 0.5 ml of human whole blood was centrifuged, resuspended in 5 ml of phosphate buffered saline (PBS) pH 7.4, centrifuged and resuspended in 2 ml of PBS. 300 $\mu$ls of this sample suspension was added to the coated cuvette and mixed. After a 2 minute incubation, the slope of the scattered light intensity was calculated over the next 8 or 18 minutes. The results were as follows:

## Slope in anti-A Coated Cuvettes

**Red Cell Phenotype**

| Sample Blood Type (RH Type) | Slope (Time) |
|---|---|
| A- | 267 ( 8 min) |
| A+ | 240 (18 min) |
| B+ | -18.6 ( 8 min) |
| O | 14.9 (18 min) |

## Slope in anti-D Coated Cuvettes

| Sample Blood Type (RH Type) | Slope (Time) |
|---|---|
| A+ | 56.6 (18 min) |
| B+ | 10.2 (18 min) |
| O-D-(high positive RH)* | 32.3 (18 min) |
| A- | 4.3 (18 min) |
| O- — | 4.5 (18 min) |

*rare blood type

It will be readily recognized by those skilled in the art that a certain amount of physical manipulation may be made to this system without substantially departing from either the spirit or the scope of the present invention. For example, the cuvettes and prism assembly may be one integral unit wherein the cuvette microtiter well is molded with a plastic prism forming part of the cuvette. Similarly, while an angle 6° above the critical angle has been found most preferred, it will be recognized that dependent upon the optical characteristics of the illumination source and the photodetector, certain variations above the critical angle may be more optimal and are to be deemed equivalent to the angle set forth herein. Further,measurements may take place on the side or bottom of the cuvette.

Further, while colloidal particles such as gold, latex and red blood cells have been described in the Examples, it should be recognized that particles and their particular size range are not to be deemed limitations but are merely exemplary of the wide range of possibilities. Indeed, the size of particles generally should be chosen with consideration given to the wavelength of the light in the liquid medium (in turn a function of the refractive index of the medium), the index of refraction of the particle should ideally be chosen with consideration given to the index of refractions of the aqueous medium and the solid so that the net effect is an optimum signal, most advantageously obtained when resonance of the system occurs. While predictability is exceedingly difficult given the current level of understanding of these complicated interactions, the actual optimization procedures are relatively simple and easily performed by those skilled in the art.

**Claims**

1. An apparatus for use in an immunoassay system employing an immunologically active component labelled with a light scattering particle for the detection of a ligand or ligand binding partner in an aqueous solution comprising;

a container for receiving the aqueous sample having a refractive index greater than that of the aqueous sample;

a light source for providing illumination;

optical coupling means for directing the illumination towards the interface between the container and the aqueous solution; and

alignment means for receiving the cuvette in fixed relationship to the optical means and the light source;

characterised in that

the illumination is directed towards the interface between the container and the aqueous solution at an angle equal to or just greater than the critical angle, and

the apparatus further comprises photodetector means positioned to receive light scattered by the light scattering particle back towards the light source.

2. An apparatus according to claim 1 wherein the illumination is directed at an angle approximately 6° greater than the critical angle.

3. An apparatus according to claim 1 or claim 2 wherein the photodetector means is arranged to receive light scattered at an angle approximately 6° greater than the critical angle.

4. An apparatus according to any one of claims 1 to 3 wherein the light source provides collimated illumination and the optical coupling means is a prism which is optically coupled to said container.

5. An apparatus according to any one of claims 1 to 4 wherein the light source is a laser.

6. An immunoassay method for the detection of a ligand or a ligand binding partner in an aqueous solution employing colloidal gold as a label in the immunoassay;

characterised in that

the immunoassay is conducted in the field of an evanescent wave of an illumination directed towards the ligand or ligand binding partner at an angle, equal to or just greater than the critical angle; and wherein the method includes the steps of

measuring light scattered by the presence of the colloidal gold particle in the evanescent field back towards the original illumination; and

correlating the colloidal gold scattered light as a function of the presence of the ligand or ligand binding partner to be determined.

7. A method for determining the presence of a ligand in an aqueous sample suspected of containing the ligand comprising the steps of:

providing a first ligand binding partner specific for the ligand in the aqueous sample, the first ligand binding partner being labelled with a particle having light scattering characteristics in the aqueous sample;

combining the first ligand binding partner and the aqueous sample with one side of an optically transmissive material thereby forming an interface between the material and the sample, the material having a plurality of second ligand binding partners bound to the material in contact with the aqueous sample and capable of binding to the ligand, the optically transmissive material having a refractive index greater than the refractive index of the aqueous sample;

illuminating the interface through the optically transmissive material at an angle equal to or just greater than the critical angle to provide total internal reflectance within the material; and

measuring the amount of light scattered from the interface back towards the original illumination, such that the amount of scattered light is a function of the amount of ligand present in the assay medium.

8. A method according to claim 7 wherein the measuring step is conducted at an angle approximately 6° greater than the critical angle.

9. A method according to claim 7 or claim 8 wherein the ligand binding partners are antibodies of monoclonal or polyclonal origin to which the ligand reacts specifically therewith.

10. A method according to any one of claims 7 to 9 wherein the ligand in the sample to be determined is an antibody and the second ligand binding partners bound to the material are antigens or haptens specifically reactive with the sample antibodies.

11. A method according to any one of claims 7 to 10 wherein the particles are colloidal gold, latex particles, glass particles, metallic particles, non-metallic particles or red blood cells.

12. A method according to any one of claims 7 to 11 wherein the illuminating step comprises irradiating the surface with light from a helium-neon laser.

13. A method according to any one of claims 7 to 12 wherein the first ligand binding partner is added to the optically transmissive material prior to adding the aqueous sample.

14. A method according to any one of claims 7 to 12 wherein the aqueous sample is added to the optically transmissive material prior to adding the first ligand binding partner.

15. A method according to any one of claims 7 to 12 wherein the aqueous sample and the first ligand binding partner are mixed prior to combining with the optically transmissive material.

16. A method according to any one of claims 7 to 15 wherein the measurement step is performed a plurality of times to derive a rate which may be correlated to the presence of ligand in the aqueous sample.

**Patentansprüche**

1. Vorrichtung zur Verwendung in einem Immunassay-System, welches eine immunologisch aktive Komponente, die mit einem Licht streuenden Partikel markiert ist, zum Nachweis eines Liganden oder Ligandenbindungspartners in einer wäßrigen Lösung einsetzt, umfassend:
   einen Behälter zum Aufnehmen der wäßrigen Probe mit einem Brechungsindex größer als dem der wäßrigen Probe;
   eine Lichtquelle zum Beleuchten;
   optische Kopplungsmittel zum Richten der Beleuchtung auf die Grenzfläche zwischen dem Behälter und der wäßrigen Lösung; und
   Ausrichtungsmittel zum Aufnehmen der Küvette in einer fixierten Beziehung zu den optischen Mitteln und der Lichtquelle;
   dadurch **gekennzeichnet** daß
   die Beleuchtung auf die Grenzfläche zwischen dem Behälter und der wäßrigen Lösung unter einem Winkel gerichtet ist, der gleich oder etwas größer als der kritische Winkel ist, und
   die Vorrichtung weiter Photodetektormittel umfaßt, die so angeordnet sind, daß sie Licht empfangen, welches von dem Licht streuenden Partikel zurück in Richtung der Lichtquelle gestreut wird.

2. Vorrichtung nach Anspruch 1, worin die Beleuchtung unter einem Winkel ausgerichtet ist, der annähernd 6° größer als der kritische Winkel ist.

3. Vorrichtung nach Anspruch 1 oder Anspruch 2, worin das Photodetektormittel so angeordnet ist, daß es Licht empfängt, welches unter einem Winkel gestreut wird, der annähernd 6° größer als der kritische Winkel ist.

4. Vorrichtung nach einem der Ansprüche 1 bis 3, worin die Lichtquelle eine kollimierte Beleuchtung bereitstellt und das optische Kopplungsmittel ein Prisma ist, welches optisch mit dem Behälter gekoppelt ist.

**5.** Vorrichtung nach einem der Ansprüche 1 bis 4, worin die Lichtquelle ein Laser ist.

**6.** Immunassayverfahren zum Nachweis eines Liganden oder eines Ligandenbindungspartners in einer wäßrigen Lösung, welches kolloidales Gold als Markierung in dem Immunassay verwendet; dadurch **gekennnzeichnet**, daß der Immunassay in dem Feld einer abklingenden Welle einer Beleuchtung durchgeführt wird, die auf den Liganden oder Ligandenbindungspartner unter einem Winkel gerichtet ist, der gleich oder etwas größer als der kritische Winkel ist; und worin das Verfahren die Schritte einschließt: Messen von Licht, welches durch die Anwesenheit des kolloidalen Goldpartikels in dem abklingenden Feld zurück in Richtung der ursprünglichen Beleuchtung gestreut wird; und das Korrelieren des vom kolloidalen Gold gestreuten Lichtes als Funktion der Anwesenheit des Liganden oder Ligandenbindungspartners, der bestimmt werden soll.

**7.** Verfahren zum Bestimmen der Anwesenheit eines Liganden in einer wäßrigen Probe, von der angenommen wird, daß sie den Liganden enthält, umfassend die Schritte: Bereitstellen eines ersten Ligandenbindungspartners, der für den Liganden in der wäßrigen Probe spezifisch ist, wobei der erste Ligandenbindungspartner mit einem Partikel markiert ist, welches Licht streuende Eigenschaften in der wäßrigen Probe hat; Kombinieren des ersten Ligandenbindungspartners und der wäßrigen Probe mit einer Seite eines optisch durchlässigen Materials, wodurch eine Grenzfläche zwischen dem Material und der Probe gebildet wird, wobei das Material eine Vielzahl von zweiten Ligandenbindungspartnern an das Material in Kontakt mit der wäßrigen Probe gebunden hat und in der Lage ist, an den Liganden zu bilden, wobei das optisch durchlässige Material einen Brechungsindex hat, der größer ist als der Brechungsindex der wäßrigen Probe; Beleuchten der Grenzfläche durch das optisch durchlässige Material unter einem Winkel, der gleich oder etwas größer ist als der kritische Winkel, um eine totale innere Reflexion innerhalb des Materials zu ergeben; und das Messen der Menge des von der Grenzfläche zurück in Richtung der ursprünglichen Beleuchtung gestreuten Lichtes, so daß die Menge des gestreuten Lichtes eine Funktion der Menge des in dem Assaymedium vorhandenen Liganden ist.

**8.** Verfahren nach Anspruch 7, worin der Meßschritt unter einem Winkel durchgeführt wird, der annähernd 6° größer als der kritische Winkel ist.

**9.** Verfahren nach Anspruch 7 oder Anspruch 8, worin die Ligandenbindungspartner Antikörper von monoklonaler oder polyklonaler Herkunft sind, mit welchen der Ligand spezifisch reagiert.

**10.** Verfahren nach einem der Ansprüche 7 bis 9, worin der zu bestimmende Ligand in der Probe ein Antikörper ist und die zweiten Ligandenbindungspartner, die an das Material gebunden sind, Antigene oder Haptene sind, die spezifisch mit den Antikörpern der Probe reagieren.

**11.** Verfahren nach einem der Ansprüche 7 bis 10, worin die partikel kolloidales Gold, Latexpartikel, Glaspartikel, metallische Partikel, nicht-metallische Partikel oder rote Blutzellen sind.

**12.** Verfahren nach einem der Ansprüche 7 bis 11, worin der Beleuchtungsschritt das Beleuchten der Oberfläche mit Licht von einem Helium-Neonlaser umfaßt.

**13.** Verfahren nach einem der Ansprüche 7 bis 12, worin der erste Ligandenbindungspartner zu dem optisch durchlässigen Material vor der Zugabe der wäßrigen Probe zugegeben wird.

**14.** Verfahren nach einem der Ansprüche 7 bis 12, worin die wäßrige Probe zu dem optisch durchlässigen Material vor der Zugabe des ersten Ligandenbindungspartners zugegeben wird.

**15.** Verfahren nach einem der Ansprüche 7 bis 12, worin die wäßrige Probe und der erste Ligandenbindungspartner vermischt werden, bevor sie mit dem optisch durchlässigen Material kombiniert werden.

**16.** Verfahren nach einem der Ansprüche 7 bis 15, worin der Meßschritt mehrmals durchgeführt wird, um eine Rate abzuleiten, die mit der Anwesenheit von Liganden in der wäßrigen Probe korreliert werden kann.

## Revendications

1. Appareil destiné à être utilisé dans un système d'essai immunologique employant un composant immunologiquement actif, marqué avec une particule dispersant la lumière, pour détecter un ligand ou un agent associé de liaison au ligand dans une solution aqueuse, comprenant:

un récipient destiné à recevoir l'échantillon aqueux, ayant un indice de réfraction supérieur à celui de l'échantillon aqueux;

une source de lumière pour éclairer;

des moyens de couplage optique pour diriger la lumière vers l'interface entre le récipient et la solution aqueuse; et

des moyens d'alignement pour recevoir la cuvette en position fixe par rapport aux moyens optiques et à la source de lumière;

caractérisé en ce que la lumière est dirigée vers l'interface entre le récipient et la solution aqueuse, selon un angle égal ou juste supérieur à l'angle critique, et en ce que l'appareil comprend en outre des moyens de photodétection disposés de façon à recevoir la lumière dispersée par la particule dipersant la lumière en retour vers la source de lumière.

2. Appareil selon la revendication 1, dans lequel la lumière est dirigée selon un angle d'environ 6° supérieur à l'angle critique.

3. Appareil selon la revendication 1 ou 2, dans lequel les moyens de photodétection, sont disposés de façon à recevoir la lumière dispersée selon un angle d'environ 6° supérieur à l'angle critique.

4. Appareil selon l'une quelconque des revendications 1 à 3, dans lequel la source de lumière, procure un éclairage collimaté, et dans lequel les moyens de couplage optique, comprennent un prisme optiquement couplé au récipient.

5. Appareil selon l'une quelconque des revendications 1 à 4, dans lequel la source de lumière, est un laser.

6. Procédé d'essai immunologique pour la détection d'un ligand ou d'un agent associé de liaison au ligand, dans une solution aqueuse, en employant de l'or colloïdal comme marqueur dans l'essai immunologique; caractérisé en ce que l'essai immunologique est effectué dans le champ d'une onde évanescente d'un éclairage dirigé vers le ligand ou l'agent associé de liaison au ligand, selon un angle égal ou juste supérieur à l'angle critique; et le procédé comprend les étapes consistant:

à mesurer la lumière dispersée par la présence de la particule d'or colloïdal dans le champ évanescent, en retour vers la lumière initiale; et

à corréler la lumière dispersée par l'or colloïdal avec la présence du ligand ou de l'agent associé de liaison au ligand destiné à être analysé.

7. Procédé de détermination de la présence d'un ligand dans un échantillon aqueux suspecté de contenir le ligand, comprenant les étapes consistant:

à introduire un premier agent associé de liaison au ligand, spécifique du ligand, dans l'échantillon aqueux, le premier agent associé de liaison au ligand, étant marqué avec une particule ayant des caractéristiques de dispersion de lumière dans l'échantillon aqueux;

à combiner le premier agent associé de liaison au ligand et l'échantillon aqueux, avec une face d'une matière transmettant la lumière, de façon à former une interface entre la matière et l'échantillon, la matière comportant plusieurs deuxièmes agents associés de liaison au ligand, liés à la matière, en contact avec l'échantillon aqueux et capables de se lier au ligand, la matière transmettant la lumière ayant un indice de réfraction supérieur à l'indice de réfraction de l'échantillon aqueux;

à éclairer l'interface à travers la matière transmettant la lumière, selon un angle égal ou, juste supérieur à l'angle critique, pour obtenir une réflexion interne totale dans la matière; et

à mesurer la quantité de lumière dispersée à partir de l'interface, en retour vers la lumière initiale, de telle façon que la quantité de lumière dispersée dépende de la quantité de ligand présente dans le milieu d'essai.

**8.** Procédé selon la revendication 7, dans lequel l'étape de mesure est effectuée selon un angle d'environ 6° supérieur à l'angle critique.

**9.** Procédé selon la revendication 7 ou 8, dans lequel les agents associés de liaison au ligand, sont des anticorps d'origine monoclonale ou polyclonale, avec lesquels le ligand réagit de manière spécifique.

**10.** Procédé selon l'une quelconque des revendications 7 à 9, dans lequel le ligand dans l'échantillon destiné à être analysé, est un anticorps, et les deuxièmes agents associés de liaison au ligand, liés à la matière, sont des antigènes ou des haptènes qui réagissent de manière spécifique avec les anticorps de l'échantillon;

**11.** Procédé selon l'une quelconque des revendications 7 à 10, dans lequel les particules consistent en or colloïdal, en particules de latex, en particules de verre, en particules métalliques, en particules non métalliques ou en hématies.

**12.** Procédé selon l'une quelconque des revendications 7 à 11, dans lequel l'étape d'éclairage, comprend l'irradiation de la surface avec de la lumière provenant d'un laser hélium-néon.

**13.** Procédé selon l'une quelconque des revendications 7 à 12, dans lequel le premier agent associé de liaison au ligand, est ajouté sur la matière transmettant la lumière avant d'ajouter l'échantillon aqueux.

**14.** Procédé selon l'une quelconque des revendications 7 à 12, dans lequel l'échantillon aqueux est ajouté sur la matière transmettant la lumière avant d'ajouter le premier agent associé de liaison au ligand.

**15.** Procpédé selon l'une quelconque des revendications 7 à 12, dans lequel l'échantillon aqueux et le premier agent associé de liaison au ligand, sont mélangés avant la combinaison avec la matière transmettant la lumière.

**16.** Procédé selon l'une quelconque des revendications 7 à 15, dans lequel l'étape de mesure est effectuée plusieurs fois pour obtenir une valeur qui puisse être corrélée avec la présence du ligand dans l'échantillon aqueux.